# EUROPÄISCHE PATENTSCHRIFT

(11) **EP 1 952 782 B1**
(45) Veröffentlichungstag und Bekanntmachung des Hinweises auf die Patenterteilung: **01.09.2010**
(21) Anmeldenummer: 07405031.1
(22) Anmeldetag: 05.02.2007
(51) Int. Cl.: A61C 19/00, A61L 2/18, A61B 19/00, A61L 2/26

(54) **Adapter zur Reiningung von medizinaltechnischen Geräten**
Adapter for cleaning medical instruments
Un adaptateur destiné au nettoyage d'appareils médicaux

(43) Veröffentlichungstag der Anmeldung: 06.08.2008
(73) Patentinhaber: Oro Clean Chemie AG, 8320 Fehraltorf (CH)
(72) Erfinder: Suter, Ursula, 8320 Fehraltorf (CH)
(74) Vertreter: Rüfenacht, Philipp Michael

(56) Entgegenhaltungen:
- EP-A1- 0 709 056
- DE-A1- 3 239 549
- US-A- 5 057 283

## Beschreibung

### Technisches Gebiet

Die Erfindung betrifft einen Adapter zur Aufbereitung einer medizinaltechnischen Vorrichtung, bevorzugt eines zahnärztlichen rotierenden Instruments, umfassend einen Fluidkanal mit einem Einlass und mindestens einem Auslass, wobei bei mindestens einem Auslass eine Kupplungseinrichtung für eine Öffnung einer medizinaltechnischen Vorrichtung vorgesehen ist. Zudem beinhaltet die Erfindung einen Gerätesatz bestehend aus dem Adapter und einem Fluidbehälter. Weiter bezieht sich die Erfindung auf die Verwendung des Adapters.

### Stand der Technik

Medizinaltechnische Vorrichtungen, die während dem Gebrauch durch Zellen, Blut, Proteine, Bakterien, physiologische Flüssigkeiten oder dergleichen verunreinigt werden, müssen nach jeder Benutzung aufwändig dekontaminiert, desinfiziert und allenfalls gepflegt werden. Nur so können heutige Hygienestandards im medizinischen Bereich garantiert werden.

Die Zahnmedizin ist im Besonderen von diesen Anforderungen betroffen. Rotierende Instrumente (Hand und Winkelstücke) für Bohrer und ähnliche Werkzeuge müssen nach jeder Verwendung am Patienten gereinigt und desinfiziert, bzw. aufbereitet werden. Hierfür existieren bereits Geräte, die diese Arbeit vollautomatisch durchführen. Ein derartiges Gerät ist beispielsweise in der US-A-5,057,283 beschrieben. Der vollautomatische Ablauf garantiert eine vollständige Reinigung und Desinfektion der rotierenden Instrumente innen und aussen und reduziert das Risiko von Keimübertragungen von Patient zu Patient. Bisher sind derartige Geräte aber aufgrund ihres Preises nur beschränkt in Industrienationen verbreitet.

Als Alternative existieren einfachere mechanische Versionen, die weitaus kostengünstiger sind. Dies sind beispielsweise spezielle Adapter, über welche Dekontaminations- oder Desinfektionssprays direkt mit den aufzubereitenden rotierenden Instrumenten verbunden werden können, um diese innenseitig zu reinigen. Derartige Vorrichtungen setzen aber eine Fachperson voraus, welche die Reinigung sehr sorgfältig nach einer mehr oder weniger aufwändigen Anleitung durchführt.

Der Nachteil bei der Verwendung von derartigen Adaptern besteht darin, dass auch diese bei der Ankopplung an die rotierenden Instrumente verunreinigt werden und folglich ebenfalls gereinigt und desinfiziert werden müssen. Dies stellt einen weiteren risikobehafteten Schritt im gesamten Reinigungs- und Desinfektionsverfahren dar.

### Darstellung der Erfindung

Aufgabe der Erfindung ist es, einen dem eingangs genannten technischen Gebiet zugehörenden Adapter für medizinaltechnische Vorrichtungen zu schaffen, welcher einfach zu verwenden ist und eine hygienisch einwandfreie Aufbereitung der medizinaltechnischen Vorrichtungen sicherstellt.

Die Lösung der Aufgabe ist durch die Merkmale des Anspruchs 1 definiert. Gemäss der Erfindung umfasst der Adapter eine Kupplungseinrichtung, die als Einwegkupplung ausgebildet ist, welche (nach dem erstmaligen Ankoppeln) beim erstmaligen Entfernen von der medizinaltechnischen Vorrichtung ein Leck im Fluidkanal des Adapters erzeugt, was die Wiederverwendung verunmöglicht und wobei die Einwegkupplung als eine oder mehrere Schnappverbindungen ausgebildet ist, wobei die Schnappverbindungen in Bereichen des Fluidkanals angebracht werden, welche eine reduzierte Wandstärke aufweisen..

Gegenüber dem Stand der Technik bietet die vorliegende Erfindung mehrere Vorteile. So wird durch die Einwegkupplung sichergestellt, dass sich ein Adapter nur einmalig zur Aufbereitung einer medizinaltechnischen Vorrichtung, wie z. B. eines rotierenden Instruments verwenden lässt. Damit wird die Notwendigkeit einer aufwändigen Reinigung und Desinfektion des Adapters nach dem Gebrauch vermieden, was das Risiko von Kreuzinfektionen vermindert. Gleichzeitig ist eine hygienische Arbeitsweise bezüglich des Adapters sichergestellt, da bei der Aufbereitung von verschmutzten medizinaltechnischen Geräten neue und saubere Adapter verwendet werden müssen. Wird ein Adapter z. B. ein zweites Mal auf eine medizinaltechnische Vorrichtung, z. B. zahnärztliches rotierendes Instrument aufgesteckt, strömt die Reinigungsflüssigkeit durch das vorhandene Leck im Adapter aus. Die kompakte Bauweise des Adapters ermöglicht zudem eine kostengünstige Herstellung.

Vorteilhafterweise können die Schnapverbindungen z. B. als strukturell verformbare Vorstände am Adapter ausgestaltet sein. Besonders geeignete Materialien für die Vorstände sind Kunststoffe. Falls die Öffnung der medizinaltechnischen Vorrichtung einen automatischen Verriegelungsmechanismus aufweist, kann in einer anderen Variante auch ein mechanisch steifer Vorstand am Adapter angebracht werden. In beiden Fällen wird erreicht, dass zwischen Adapter und medizinaltechnischer Vorrichtung durch einfaches Zusammenschieben der beiden Teile eine mechanisch stabile und dichte Verbindung erhalten wird.

Die Schnappverbindungen werden bevorzugt als keilförmige Verrastnasen ausgebildet, welche vorzugsweise in einem Winkel verschieden von 90° vom Fluidkanal abstehen und besonders bevorzugt in Richtung des Einlasses des Adapters geneigt sind. Damit wird erreicht, dass sich die Verrastnasen in seitlichen Aussparungen der Öffnung der medizinaltechnischen Vorrichtung verhaken können. Durch diesen Verrastmechanismus entsteht eine mechanisch feste und fluiddichte Verbindung zwischen Adapter und Instrumentenöffnung, welche nur durch eine irreversible Beschädigung des Adapters wieder gelöst werden kann.

Die Schnappverbindungen werden in Bereichen mit reduzierter Wandstärke des Fluidkanals, welche als Sollbruchstellen vorgesehen sind, angebracht. Beim Abkuppeln des Adapters von der medizinaltechnischen Öffnung verhakt die Schnappverbindungen im Instrument und wird augrund ihrer Neigung umgeknickt was beim Erreichen der Belastungsgrenze des Materials des Fluidkanals zur Leckbildung - z. B. in Form eines Bruchs oder eines Risses - an der Sollbruchstelle des Fluidkanals führt.

Bevorzugt werden am Adapter Dichtlippen entsprechend der Form der Öffnung der medizinaltechnischen Vorrichtung angebracht. Dadurch wird eine fluiddichte Verbindung zwischen Adapter und medizinaltechnischer Vorrichtung ermöglicht. Derartige Dichtlippen können kreisförmig, oval, rechteckig oder von anderer geeigneter Form sein, welche ein fluiddichtes Verschliessen der Öffnung der medizinaltechnischen Vorrichtung ermöglicht. Das Anbringen der Dichtlippen direkt am Adapter hat den Vorteil, dass beim Aufstecken des Adapters auf die medizinaltechnische Vorrichtung die erwünschte Abdichtung der Verbindung automatisch erreicht wird.

Da viele der heute verbreiteten medizinaltechnischen Vorrichtungen und insbesondere zahnärztliche Hand- und Winkelstücke intern mehrere Fluidkanäle, wie z. B. Flüssigkeits-oder Luftkanäle aufweisen, sind Adapter mit zwei oder mehreren Auslässen von besonderem Vorteil. Die unterschiedlichen Auslässe erlauben die gleichzeitige Reinigung der verschiedenen Hohlräume (Spraykanäle und Getriebekanal), was einer Vereinfachung und Verkürzung des gesamten Reinigungsvorgangs gleichkommt. Aufgrund des Aufbaus von rotierenden Instrumenten von besonderem Vorteil sind Adapter bei denen mindestens ein Auslass quer zu einer Längsrichtung des Fluidkanals und wenigstens ein Auslass entlang der Längsrichtung des Fluidkanals ausgebildet sind. Möglich sind aber auch andere Adapter mit mehreren Kanälen, die ausschliesslich quer oder entlang der Längsrichtung des Adapters verlaufen.

Weist ein Adapter mehrere Auslässe auf, werden die Querschnittsflächen der einzelnen Auslassöffnungen in einer vorteilhaften Variante entsprechend der Geometrie des Instrumentes und besonders vorteilhaft zudem unterschiedlich gross ausgebildet. Das Verhältnis einer Querschnittsfläche einer längsgerichteten Auslassöffnung zu einer Querschnittsfläche einer quergerichteten Auslassöffnung weist dabei bevorzugt einen Wert im Bereich von 30 - 140 auf. Dadurch wird erreicht, dass aus den verschiedenen Auslässen unterschiedliche Fluidmengen fliessen. Die damit ermöglichte gezielte Fluidverteilung hat den wesentlichen Vorteil, dass die Reinigungswirkung in den verschiedenen Hohlräumen der medizinaltechnischen Vorrichtung dem Grad der Verunreinigung angepasst und optimiert werden kann.

Am Adapter können zwischen den Auslässen zudem Vorstände oder Lippen angebracht werden, welche die Öffnung der medizinaltechnischen Vorrichtung in fluiddicht voneinander separierte Teile unterteilt. Diese Vorstände oder Lippen werden vorzugsweise der jeweiligen Querschnittsgeometrie der Öffnung der medizinaltechnischen Vorrichtung an der Position der Vorstände oder Lippen angepasst. Besonders interessant sind kreisförmige Öffnungen, da diese ideal für Bohrungen geeignet sind. Durch die fluiddichte Trennung der einzelnen Auslässe ist es möglich, die zur Reinigung verwendeten Fluide gezielt in unterschiedliche Bereiche der medizinaltechnischen Vorrichtungen zu verteilen und allenfalls gewisse Bereiche der Instrumente vor Fluid zu schützen.

Ferner können im Fluidkanal Verengungen zur Regulierung des Fluidstroms durch den Adapter angebracht werden. Diese können runde, ovale oder rechteckige Querschnitte aufweisen und von unterschiedlicher Länge sein. Damit wird erreicht, dass ein allenfalls zu starker Fluidstrom im Einlassbereich des Adapters im Bereich der Auslassöffnungen wesentlich reduziert werden kann und somit die Kupplungsvorrichtung des Adapters gegen Überlast durch eine zu grosse Druckbelastung geschützt wird.

Im Bereich des Einlasses des Adapters kann zudem eine Verbindungsvorrichtung angebracht werden. Grundsätzlich sind alle aus der Verbindungstechnik bekannten Vorrichtungen verwendbar. Vorzugsweise werden Gewinde, Komponenten für Bajonettverschlüsse, Komponenten für Luer-Verschlüsse, Klammern oder Vorsprünge angebracht. Dies hat den Vorteil, dass sich der Adapter auf der Seite des Einlasses mit unterschiedlichsten Fluidbehältern wie z. B. Spritzen, Spraydosen oder fluidführenden Leitungen verbinden lässt.

Der Adapter wird bevorzugt vollständig aus nur einem Werkstoff gefertigt. Insbesondere geeignet hierfür sind Kunststoffe wie z. B. Polypropylen oder ähnliche Materialien. Dadurch lassen sich die Herstellungskosten tief halten und die Anforderung an die chemische Stabilität des Adapters wird dennoch erfüllt.

Am Adapter seitlich angebrachte Haltevorsprünge zum Ziehen und Stossen erleichtern das Zusammenfügen und das Trennen des Adapters und der medizinaltechnischen Vorrichtung. Die Haltevorsprünge stehen bevorzugt quer zur Längsrichtung des Fluidkanals vom Adapter weg und können rechteckig, elliptisch, trapezförmig oder sonst geeignet geformt sein, um beim An- bzw. Abkoppeln des Adapters von Hand genügend Stoss- bzw. Zugkraft auf den Adapter zu übertragen. Der Vorstand der Haltevorsprünge vom Adapter ist von Vorteil grösser als 4 mm, wodurch ein Abrutschen der Finger beim Ziehen und Stossen verhindert wird.

Zum Aufbereiten wird über den Einlass des Adapters ein Fluid, z. B. ein flüssiges Reinigungs- oder Desinfektionsmittel, durch den Adapter in die zu reinigenden und zu desinfizierenden Hohlräume der angekoppelten medizinaltechnischen Vorrichtung geleitet.

Hierfür wird der Einlass des Adapters mit einem Fluidbehälter bestückt, der von Vorteil eine Vorrichtung zum Druckaufbau im Fluidbehälter beinhaltet. Durch die Vorrichtung zum Druckaufbau lässt sich das im Behälter enthaltene Fluid in einfacher Weise in den Adapter entleeren. In der Praxis haben sich speziell Spritzen oder Spraydosen als ideal erwiesen, da diese aufgrund ihrer Grösse gut handhabbar sind. Spraydosen bieten zudem den Vorteil, dass abgesehen von der einfachen Handhabung, aufgrund der kontinuierlichen Fluidabgabe bei konstantem Druck eine präzise Dosierung der Reinigungsmittel erreicht wird. Es können aber auch grössere Fluidbehälter wie Vorratsflaschen oder Kanister verwendet werden, die z. B. auch über eine Fluidleitung mit dem Adapter verbunden werden können. In diesen Fällen eignen sich zum Druckaufbau besonders Pumpen, die in die Fluidbehälter oder -leitungen integriert werden können.

Von besonderem Interesse ist die Verwendung des erfindungsgemässen Adapters im Zusammenhang mit der Aufbereitung von zahnärztlichen Hand- und Winkelstücken. Durch die geringen Abmessungen des Adapters mit angekoppeltem Instrument und Spritze als Fluidbehälter, lässt sich der gesamte Gerätesatz bequem von einer einzelnen Person handhaben.

Bevorzugt werden einzelne Aufbereitungsvorgänge mehrmals mit unterschiedlichen Fluiden wiederholt. Damit können medizinaltechnische Vorrichtungen von verschiedensten Kontaminationen wie z. B. Blut, Proteinen, menschlichen Zellen oder Mikroorganismen befreit werden. Ein typischer Aufbereitungsvorgang sieht folgendermassen aus:
1. Eine Spritze als Flüssigkeitsbehälter wird mit einer Reinigungslösung bestückt.
2. Das Adapterstück wird an die medizinaltechnische Vorrichtung, z. B. ein rotierendes Instrument angekoppelt.
3. Die Spritze wird am Einlass des Adapters aufgesetzt.
4. Der Kolben der Spritze wird in die Spritze gedrückt und dabei baut sich ein Überdruck auf, welcher die Reinigungslösung durch den Adapter und die zu reinigenden Hohlräume der medizinaltechnischen Vorrichtung fliessen lässt.
5. Die Spritze wird vom Adapter entfernt und mit Luft gefüllt.
6. Die mit Luft gefüllte Spritze wird wieder auf den Adapter aufgesetzt und entleert, wodurch Reste der Reinigungslösung aus dem Adapter und der medizinaltechnischen Vorrichtung ausgestossen wird.
7. Die Schritte 2-6 werden bevorzugt mit Desinfektionslösungen oder anderen Lösungen wiederholt.
8. Das Adapterstück wird von der medizinaltechnischen Vorrichtung entfernt und bricht auf, wodurch es unbrauchbar wird.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Querschnitt durch einen erfindungsgemässen Adapter.
- Fig. 2: Ansicht des Auslassbereichs des Adapters aus Fig. 1 entlang des Fluidkanals.
- Fig. 3: Dreidimensionale Darstellung einer Variante des Adapters aus Fig. 1.
- Fig.4: Querschnitt durch die Öffnung eines zahnärztlichen rotierenden Instruments
- Fig. 5: Ansicht in die Öffnung eines zahnärztlichen rotierenden Instruments aus Fig. 4.
- Fig. 6: Adapter gekoppelt an ein zahnärztliches rotierendes Instrument.
- Fig. 7: Adapter nach der Entkoppelung vom zahnärztlichen rotierenden Instrument.
- Fig. 8: Adapter gekoppelt an ein zahnärztliches rotierendes Instrument und einen Flüssigkeitsbehälter.

Grundsätzlich sind in den Figuren gleiche Teile mit gleichen Bezugszeichen versehen.

### Wege zur Ausführung der Erfindung

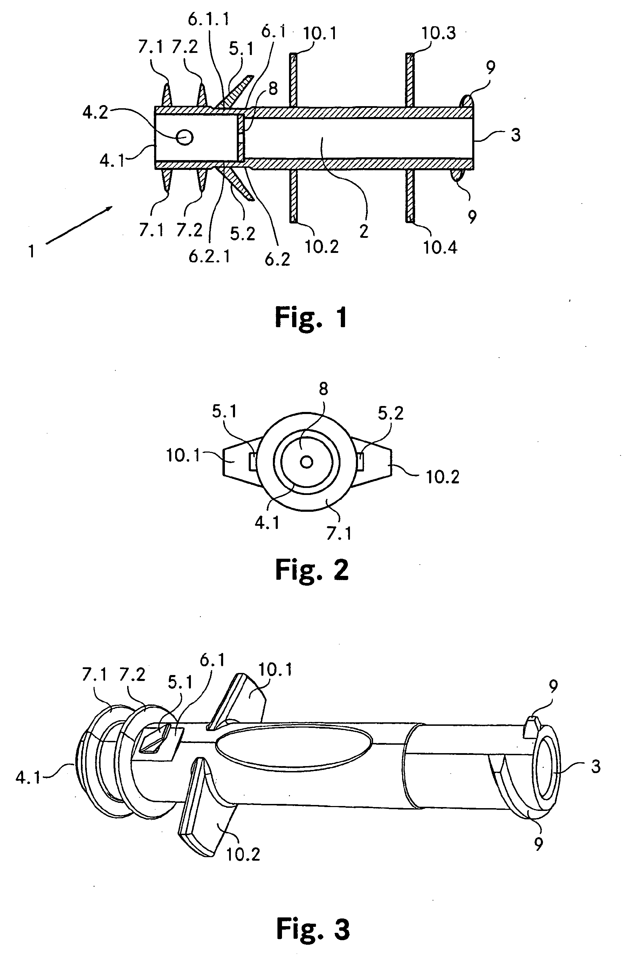

Ein in Fig. 1 und 2 gezeigter Adapter 1 für eine medizinaltechnische Vorrichtung umfasst einen zylindrischen Fluidkanal 2, mit einem Einlass 3, einem ersten Auslass 4.1 entlang der Richtung des Fluidkanals und einem zweiten, quer dazu stehenden Auslass 4.2. Der Adapter 1 ist vollständig aus Polypropylen gefertigt. Die Aussenseite des Fluidkanals 2 weist im Bereich der Auslässe 4.1, 4.2 zwei rechteckige Abflachungen 6.1, 6.2 an der Aussenseite des zylindrischen Fluidkanals 2 auf, welche je eine Kopplungsvorrichtung in Form von zwei Verrastnasen 5.1, 5.2 für eine Schnappverbindung tragen. Die Wandstärke 6.1.1, 6.2.1 des Fluidkanals 2 ist aufgrund der Abflachungen 6.1, 6.2 an der Befestigungsfläche der Verrastnasen geringer als in den anderen Bereichen des Fluidkanals 2. Die Verrastnasen 5.1, 5.2 sind keilförmig ausgestaltet und mit ihrem breiten Ende mit den Abflachungen 6.1, 6.2 verbunden, wobei sie in Richtung des Auslasses 3 geneigt, in einem Winkel von beispielsweise 50° von der Aussenseite des Fluidkanals abstehen. Vor und nach dem querstehenden Auslass 4.2 sind zwei als Ringflansche ausgebildete Dichtlippen 7.1, 7.2 an der Aussenseite des Fluidkanals 2 angebracht. Diese umschliessen den Adapter 1 aussen vollständig und sind als Ringe ausgebildet. Die Dicke der Dichtlippen 7.1, 7.2 im Querschnitt ist direkt an der Aussenseite des Fluidkanals 2 am grössten und nimmt in der Richtung weg vom Fluidkanal stetig ab. Der äussere Durchmesser der Dichtlippen 7.1, 7.2 ist so gewählt, dass die radialen Vorstände der Dichtlippen kleiner sind als die Vorstände der Verrastnasen 5.1, 5.2.

Im Innern des Fluidkanals 2 ist eine ringförmige Verengung 8 angebracht, die den Fluidkanal 2 in einen vorderen Teil hin zum Auslassbereich und einen hinteren Teil hin zum Einlassbereich aufteilt. Die beiden Teile weisen unterschiedliche Durchmesser auf. Im Bereich des Einlasses 3 ist eine Verbindungsvorrichtung 9 in Form eines Gewindes angebracht. Im Bereich zwischen Einlass 3 und Verrastnasen 5.1, 5.2 sind an der Aussenseite des Fluidkanals 2 mehrere Haltevorsprünge 10.1, 10.2, 10.3, 10.4 angebracht, welche senkrecht zum Fluidkanal 2 stehen und eine trapezähnliche Oberfläche aufweisen.

Für handelsübliche zahnärztliche rotierende Instrumente weisen die Dichtlippen 7.1, 7.2 einen Aussendurchmesser von z. B. 10 mm auf, während der Innendurchmesser des längsgerichteten Auslasses 4.1 z. B. 5.8 mm und der des quer angebrachten Auslasses 4.2 z. B. 0.5 - 1 mm beträgt. Die Länge des gesamten Adapters 1 beträgt z. B. 48 mm und die Haltevorsprünge 10.1, 10.2, 10.3, 10.4 stehen bevorzugt mindestens 4 mm von der Aussenseite des Fluidkanals 2 ab.

Fig. 3 zeigt ein Ausführungsbeispiel eines Adapters 1, welcher nur zwei axial gegenüberliegende Haltevorsprünge 10.1, 10.2 aufweist, die zudem senkrecht zur Richtung der Verrastnasen 5.1, 5.2 angebracht sind. Zudem ist die Aussenseite des Fluidkanals uneben ausgestaltet, was für die Handhabung des Adapters 1 hilfreich ist.

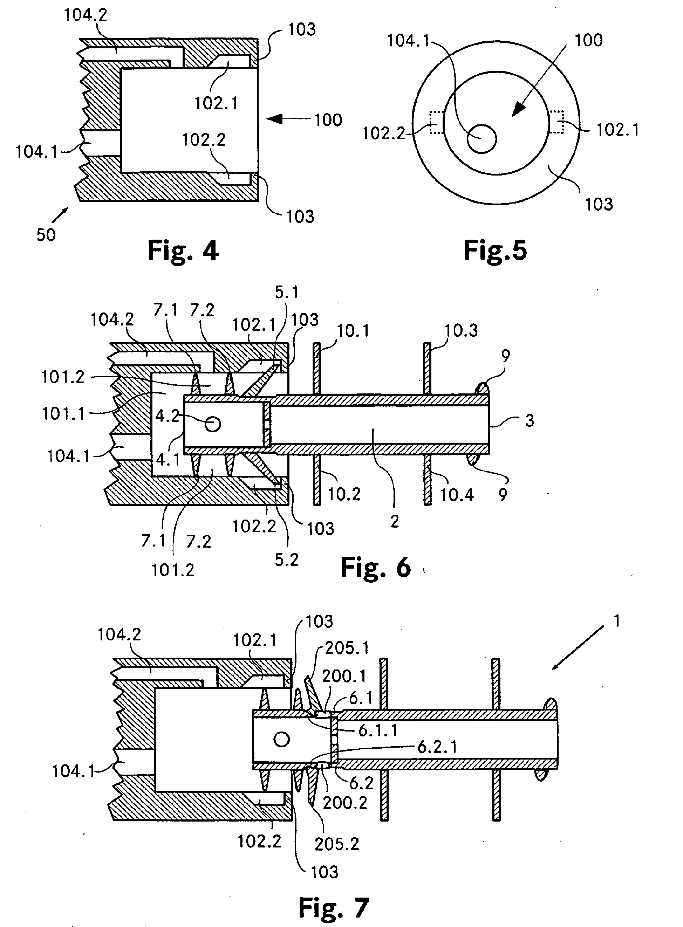

Die Öffnung 100 der medizinaltechnischen Vorrichtung 50 kann beispielsweise eine Anschluss-Kupplung eines rotierenden Instruments für den Betrieb von Bohrern und ähnlichen rotierenden Werkzeugen sein. Derartige Instrumente verfügen z. B. über einen Getriebe- und Spraykanal, welche während dem Gebrauch durch Zellen, Blut, Proteine, Mikroorganismen und physiologische Flüssigkeiten verschmutzt werden. In Fig. 4 und 5 ist ein Beispiel einer Öffnung 100 eines zahnärztlichen rotierenden Instruments gezeigt. Diese besteht aus einer Bohrung mit einem Durchmesser, welcher dem radialen Durchmesser der Dichtlippen 7.1, 7.2 des Adapters 1 entspricht. Zudem sind zwei Vertiefungen 102.1, 102.2 am Beginn der Bohrung vorhanden, welche aufgrund ihrer Grösse die Aufnahme der Verrastnasen 5.1, 5.2 in verbiegungsfreiem Zustand ermöglichen.

Beim Einschieben des Adapters 1 unter Verwendung der Haltevorsprünge 10.1, 10.2, 10.3, 10.4 in die Öffnung 100 des rotierenden Instruments 50 werden die Verrastnasen 5.1, 5.2 an der Stirnseite 103 der Bohrung elastisch zusammengedrückt, bis sie nach vollständigem Einschieben im Bereich der Vertiefungen 102.1, 102.2 sich entfalten und in verbiegungsfreiem Zustand vorliegen. Dadurch schnappt der Adapter 1 wie in Fig. 6 gezeigt in der Öffnung 100 der medizinaltechnischen Vorrichtung 50 ein, wobei aufgrund der durch die Stirnseite 103 begrenzten Vertiefungen 102.1, 102.2 eine mechanisch stabile Verbindung entsteht. Die Dichtlippen 7.1, 7.2 teilen die Bohrung in ein erstes Teilvolumen 101.1, welches mit einem ersten Fluidkanal 104.1 korrespondiert und ein zweites Teilvolumen 101.2, welches mit einem zweiten Fluidkanal 104.2 des rotierenden Instruments korrespondiert. Die Fluidkänale 104.1, 104.2 führen aus der Öffnung 100 in weitere, nicht gezeigte Bereiche des rotierenden Instruments 50.

In Fig. 7 ist das Herausziehen bzw. Abkoppeln des Adapters 1 aus der Öffnung 100 des rotierenden Instruments 50 gezeigt. Dabei verhaken sich die Verrastnasen an der Stirnseite 103 der Bohrung, wodurch im Bereich der Abflachungen 6.1, 6.2 aufgrund der Neigung der Verrastnasen 5.1, 5.2 ein seitlicher Druck auf die Wand des Fluidkanals entsteht, welcher beim Erreichen der Belastungsgrenze des Materials zum Bruch des Fluidkanals 2 führt. Dabei entstehen aufgrund der reduzierten Wandstärken 6.1.1, 6.2.1 im Bereich der Abflachungen 6.1, 6.2 irreversibel zwei leckartige Öffnungen 200.1, 200.2 im Fluidkanal 2, wobei die Verrastnasen 205.1, 205.2 in die Gegenrichtung geknickt werden.

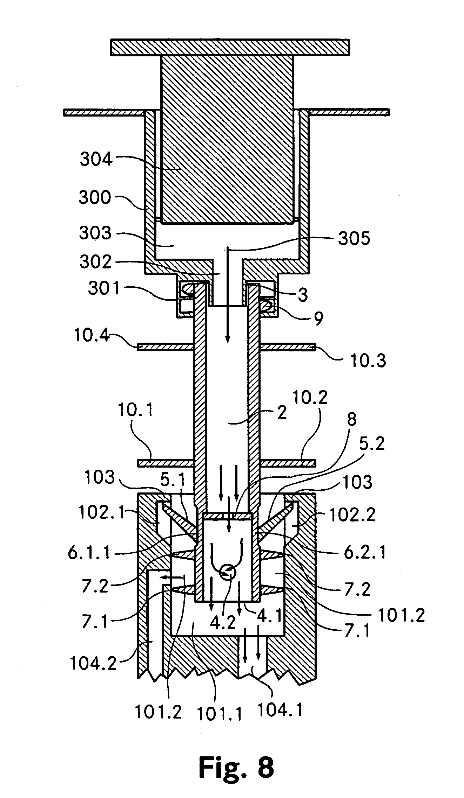

Zur Reinigung eines rotierenden Instumentes 50 wird der Auslassbereich des Adapters 1 in die Öffnung 100 des rotierenden Instruments 50 eingeschoben und am Einlass 3 des Adapters 1 ein Fluidreservoir mit einem Reinigungsmittel aufgesetzt. Im Ausführungsbeispiel, welches in Fig. 8 gezeigt ist, wird eine Spritze 300 mit einem Innengewinde 301 auf das Aussengewinde 9 des Adapters 1 geschraubt, wodurch die Spritze 300 kraftschlüssig und fluiddicht mit dem Adapter 1 verbunden wird. Dabei mündet der Auslass der Spritze 302 direkt in den Fluidkanal 2. Im Fluidvolumen 303 der Spritze 300 befindet sich ein geeignetes Fluid 305, z. B. ein Reinigungs- oder Desinfektionsmittel, welches insbesondere flüssig vorliegt. Durch Druck auf den Kolben der Spritze 304 wird das Fluid 305 in den Fluidkanal 2 gepresst und gelangt nach Drosselung des Fluidstroms durch die Verengung 8 zu den Auslässen 4.1, 4.2 des Adapters 1. Damit eine fluiddichte Verbindung zwischen Adapter 1 und Öffnung 100 des rotierenden Instruments 50 vorliegt, müssen die Verrastnasen 5.1, 5.2 in den Vertiefungen 102.1, 102.2 verrastet sein. Ansonsten wird der Adapter 1 durch den Fluiddruck wieder aus der Öffnung 100 des Instruments 50 heraus geschoben. Aufgrund der geometrisch unterschiedlich angeordenten und unterschiedlich grossen Querschnittsflächen der Auslässe 4.1, 4.2 fliessen verschieden starke Fluidströme in die Teilvolumen 101.1, 101.2 und von da aus weiter in die Hohlräume 104.1, 104.2 der Öffnung 100 des Instruments 50. Dadurch werden die Fluidkanäle 104.1, 104.2 sowie nicht gezeigte weiter innen liegende Bereiche des Instruments 50 durch das Fluid 305 gereinigt. Die gezielte Steuerung der Stärke der Fluidströme durch die unterschiedlich dimensionierten Auslässe 4.1, 4.2 ist besonders vorteilhaft, wenn beispielsweise der zweite Kanal 104.2 des rotierenden Instruments verglichen mit dem ersten Kanal 104.1 nur leicht verschmutzt ist. Aufgrund der kleineren Querschnittsfläche und der geometrisehen Anordnung des zweiten Auslasses 4.2 verglichen mit dem ersten Auslass 4.1, fliesst eine geringere Menge an Reinigungsmittel durch den Kanal 104.2, was den Verbrauch deutlich reduziert.

Zur Aufbereitung von zahnärztlichen rotierenden Instrumenten beispielsweise, werden bevorzugt mehrere Reinigungsschritte nacheinander ausgeführt: Zuerst wird der Adapter 1 in die Öffnung 100 des Instruments gekoppelt, so dass die Verrastnasen 5.1, 5.2 in den Vertiefungen 102.1, 102.2 verrasten. Dann wird eine Spritze 300, bestückt mit einer Reinigungslösung, auf den Einlass 3 des Adapters geschraubt. Das Reinigungsmittel wird danach durch Druck auf den Kolben 304 der Spritze in den Fluidkanal 2 des Adapters 1 geleitet und wie vorstehend beschrieben weiter durch die zu reinigenden Fluidkanäle 104.1, 104.2 des Instruments. Danach wird die Spritze 300 mit dem Reinigungsmittel vom Einlass 2 des Adapters 1 abgeschraubt und die Spritze 300, gefüllt mit Luft auf den Einlass 2 des Adapters 1 geschraubt. Analog zum Reinigungsmittel wird auch die Luft durch die Fluidkanäle 104.1, 104.2 des zahnärztlichen Instruments geleitet. Dabei werden die Rückstände des Reinigungsmittels aus dem vorangehenden Reinigungsschritt aus der Öffnung und der Fluidkanäle 104.1, 104.2 des Instruments entfernt. Danach wird die Spritze 300, vom Einlass 2 des Adapters 1 entfernt und bestückt mit einem Desinfektionsmittel wieder auf den Einlass 2 des Adapters 1 aufgeschraubt. Analog zum Reinigungsmittel wird auch das Desinfektionsmittel durch die Fluidkanäle 104.1, 104.2 des zahnärztlichen Handstücks geleitet und danach wird die Spritze 300 entfernt. Anschliessend wird mit der Spritze in analoger Weise Luft durch die Öffnung 100 und die Fluidkanäle 104.1, 104.2 des rotierenden Instruments. geleitet und Reste des Desinfektionsmittels ausgeblasen. Nach erfolgter Reinigung wird der Adapter 1 aus der Öffnung der medizinaltechnischen Vorrichtung herausgezogen, wobei der Adapter wie vorstehend beschrieben und in Fig. 7 gezeigt, aufbricht und unbrauchbar wird.

In anderen Ausführungsformen kann der Adapter 1 einen gekrümmten oder abgewinkelten Fluidkanal 2 aufweisen und beispielsweise auch die Form eines ovalen oder rechteckigen Hohlprofils aufweisen. Insbesondere können auch zusätzliche Vorstände oder Kanten am Adapter 1 zur definierten Orientierung relativ zur medizinaltechnischen Vorrichtung angebracht sein. Der Fluidkanal 2 kann über seine gesamte Länge von konstantem Innendurchmesser sein oder verschiedene Bereiche mit unterschiedlichen Innendurchmessern aufweisen und mehrere Verengungen enthalten. Die Anzahl der Dichtlippen 7.1, 7.2 und der Verbindungsvorrichtungen 5.1, 5.2 hängt von der Ausgestaltung der medizinaltechnischen Vorrichtung ab, und kann entsprechend variieren. Es können insbesondere mehr als zwei Auslässe 4.1, 4.2 vorhanden sein, die in beliebiger Richtung relativ zur Längsrichtung des Fluidkanals 2 verlaufen. Insbesondere können die als Sollbruchstellen dienenden Bereiche mit reduzierten Wandstärken 6.1.1, 6.2.1 im Fluidkanal 2 auch als ringförmige Verjüngung im gesamten Umfang des Fluidkanals 2 oder als schlitzartige Einkerbungen ausgestaltet sein.

Im Bereich des Einlasses 3 kann anstelle des Gewindes 9 auch eine Komponente eines Bajonettverschlusses, eine Komponente eines Luer-Verschlusses, eine Klammer oder ein Vorsprung angebracht sein. Die geometrischen Formen des Einlasses sowie der Auslässe können unterschiedlich sein und Asymmetrien, Ecken oder Abflachungen der Kanten aufweisen. Ebenso können die Haltevorsprünge 10.1, 10.2, 10.3, 10.4 je nach Ausgestaltung des Adapters 1 an anderen Stellen und in unterschiedlicher Anzahl am Adapter angebracht sein, um die Handhabung beim An- und Abkoppeln der medizinaltechnischen Vorrichtung 50 zu optimieren.

Es sind auch andere Ausgestaltungen der Öffnung 100 der medizinaltechnischen Vorrichtung 50 möglich. Insbesondere können mehr als zwei Fluidkanäle 104.1, 104.2 aus der Öffnung in innere Bereiche der medizinaltechnischen Vorrichtung führen. Auch andere, nicht runde Geometrien mit asymmetrischen Aussparungen, die eine definierte Orientierung des Adapters 1 bezüglich der Rotation um die Achse der Öffnung 100 der medizinaltechnischen Vorrichtung 50 sicherstellen, sind möglich. Der Adapter 1 wird in diesen Fällen auf die vorliegende Öffnung der medizinaltechnischen Vorrichtung 50 entsprechend angepasst.

In einer anderen vorteilhaften Ausführungsform kann anstelle einer Spritze 300 ein Schlauch an den Einlass 3 des Adapters gekoppelt werden, welcher den Adapter 1 über eine Pumpe mit einer Vorratsflasche oder einem Kanister verbindet. Auch unter Druck stehende Spraydosen können insbesondere als Fluidreservoir dienen und über einen Schlauch an den Einlass 3 des Adapters 1 angeschlossen werden. Ebenso können anstelle von Gewinden als Verbindungsvorrichtungen 9, 301 andere Verbindungselemente wie z. B. Klammern, Vorstände oder Komponenten von Bajonettverschlüssen am Adapter 1 und am Fluidreservoir angebracht werden.

Je nach medizinaltechnischer Vorrichtung 50 kann es vorteilhaft sein, zusätzliche Reinigungsmittel zu verwenden, die spezifisch bestimmte Verunreinigungen lösen. Diese können insbesondere als Flüssigkeiten, Gase oder Aerosole vorliegen. Anstatt Luft kann natürlich auch jedes andere gasförmige Fluid, z. B Stickstoff oder Edelgase zum Ausblasen der Reinigungs- bzw. Desinfektionsmittel aus der Öffnung 100 und den Fluidkanälen 104.1, 104.2 der medizinaltechnischen Vorrichtung verwendet werden.

Zusammenfassend ist festzustellen, dass ein neuer Adapter zur Verbindung eines Fluidreservoirs mit der Öffnung einer medizinaltechnischen Vorrichtung, wie z. B. rotierender Instrumente entwickelt wurde, welcher eine hygienisch einwandfreie Instrumentenaufbereitung sicherstellt. Der Adapter basiert auf einer Verbindungsvorrichtung die beim Entkoppeln des Adapters von der Öffnung der medizinaltechnischen Vorrichtung zum Bruch des Adapters führt. Damit kann ein Adapter immer nur zur Reinigung einer einzigen medizinaltechnischen Vorrichtung verwendet werden. Die erfindungsgemässen Adapter sind prinzipiell für alle medizinaltechnischen Vorrichtungen, welche Öffnungen im Gehäuse aufweisen, verwendbar.

## Patentansprüche

1. Adapter (1) zur Aufbereitung einer medizinaltechnischen Vorrichtung bevorzugt eines zahnärztlichen rotierenden Instruments, umfassend einen Fluidkanal (2) mit einem Einlass (3) und mindestens einem Auslass (4.1, 4.2), wobei bei dem mindestens einen Auslass eine Kupplungseinrichtung (5.1, 5.2) für eine Öffnung (100) der medizinaltechnischen Vorrichtung (50) vorgesehen ist, **dadurch gekennzeichnet, dass** die Kupplungseinrichtung (5.1, 5.2) als Einwegkupplung ausgebildet ist, welche beim erstmaligen Entfernen von der medizinaltechnischen Vorrichtung (50) ein Leck (200.1, 200.2) im Fluidkanal (2) des Adapters (1) erzeugt und wobei die Einwegkupplung als eine oder mehrere Schnappverbindungen ausgebildet ist, wobei die Schnappverbindungen (5.1, 5.2) in Bereichen des Fluidkanals (2) angebracht werden, welche eine reduzierte Wandstärke (6.1.1, 6.2.1) aufweisen.

2. Adapter (1) nach Anspruch 1, **dadurch gekennzeichnet, dass** die Schnappverbindungen strukturell biegbar sind und besonders bevorzugt aus Kunststoff bestehen.

3. Adapter (1) nach Anspruch 2, **dadurch gekennzeichnet, dass** die Schnappverbindungen (5.1, 5.2) als keilförmige Verrastnasen ausgebildet sind, die bevorzugt in einem Winkel verschieden von 90° vom Fluidkanal (2) abstehen und besonders bevorzugt in Richtung zum Einlass (3) geneigt sind.

4. Adapter (1) nach einem der Ansprüche 1-3, **dadurch gekennzeichnet, dass** am Adapter Dichtlippen (7.2) von kreisförmiger, ovaler oder rechteckiger Form, zum fluiddichten Verschliessen der Öffnung der medizinaltechnischen Vorrichtung angebracht sind.

5. Adapter (1) nach einem der Ansprüche 1-4, **dadurch gekennzeichnet, dass** der Adapter (1) zwei oder mehrere Auslässe (4.1, 4.2) beinhaltet und bevorzugt mindestens ein Auslass (4.2) quer zu einer Längsrichtung des Fluidkanals (2) und wenigstens ein Auslass (4.1) entlang der Längsrichtung des Fluidkanals (2) ausgebildet ist.

6. Adapter (1) nach einem der Ansprüche 15, **dadurch gekennzeichnet, dass** bei mehreren Auslässen (4.1, 4.2) Querschnittsflächen der Auslassöffnungen unterschiedlich gross sind, wobei ein Verhältnis einer Querschnittsfläche einer längsgerichteten Auslassöffnung zu einer Querschnittsfläche einer quergerichteten Auslassöffnung bevorzugt einen Wert im Bereich von 30 - 140 aufweist.

7. Adapter (1) nach einem der Ansprüche 1-6, **dadurch gekennzeichnet, dass** im Fluidkanal (2) eine Verengung (8) zur Drosselung des Fluidstroms durch den Adapter (1) angebracht ist.

8. Adapter (1) nach einem der Ansprüche 5-7, **dadurch gekennzeichnet, dass** am Adapter (1) zwischen den Auslässen (4.1, 4.2) Vorstände oder Lippen (7.1, 7.2) angebracht sind, zur Unterteilung der Öffnung (100) der medizinaltechnischen Vorrichtung (50) in fluiddicht voneinander separierte Teile (101.1, 101.2).

9. Adapter (1) nach einem der Ansprüche 1-8, **dadurch gekennzeichnet, dass** in einem Bereich des Einlasses (3) des Adapters (1) eine Verbindungsvorrichtung (9) angebracht ist, vorzugsweise ein Gewinde, eine Komponente eines Bajonettverschlusses, eine Komponente eines Luer-Verschlusses, eine Klammer oder ein Vorsprung.

10. Adapter (1) nach einem der Ansprüche 1-9, **dadurch gekennzeichnet, dass** der Adapter (1) vollständig aus nur einem Werkstoff gefertigt wird, welcher bevorzugt Kunststoff und besonders bevorzugt Polypropylen ist.

11. Adapter (1) nach einem der Ansprüche 1-10, **dadurch gekennzeichnet, dass** am Adapter (1) seitliche Haltevorsprünge (10.1, 10.2, 10.3, 10.4) zum Ziehen und Stossen angebracht sind, die das Zusammenfügen und das Trennen des Adapters (1) und der medizinaltechnischen Vorrichtung (50) erleichtern, wobei der Vorstand der Haltevorsprünge (10.1, 10.2, 10.3, 10.4) von der Aussenseite des Adapters (1) bevorzugt mehr als 4 mm beträgt.

12. Gerätesatz umfassend eine medizinaltechnische Vorrichtung (50), einen Adapter (1) nach einem der Ansprüche 1-11 und einen Fluidbehälter (300) **dadurch gekennzeichnet, dass** der Einlass (3) des Adapters (1) fluiddicht mit dem Fluidbehälter (300) verbunden ist, welcher bevorzugt über eine Vorrichtung zum Druckaufbau (304) verfügt, wie insbesondere bei Spritzen oder Spraydosen vorhanden, und dass gleichzeitig die Auslässe (4.1, 4.2) des Adapters (1) fluiddicht an die medizinaltechnische Vorrichtung, welche bevorzugt ein zahnärztliches rotierendes Instrument (50) ist, gekoppelt sind.

13. Verwendung eines Adapters (1) nach einem der Ansprüche 1-11, **dadurch gekennzeichnet, dass** über den Einlass (3) des Adapters (1) ein Flujd (305) durch den Adapter (1) in die zu reinigenden Kanäle (104.1, 104.2) einer angekoppelten medizinaltechnischen Vorrichtung (50) geleitet wird.

## Claims

1. Adapter (1) for treatment of a medical device, preferably a dental rotary instrument, comprising a fluid channel (2) with an inlet (3) and at least one outlet (4.1, 4.2), the at least one outlet being provided with a coupling means (5.1, 5.2) for an opening (100) of the medical device (50), **characterized in that** the coupling means (5.1, 5.2) is designed as a single-use coupling which, when removed for the first time from the medical device (50), generates a leak (200.1, 200.2) in the fluid channel (2) of the adapter (1), and wherein the single-use coupling is designed as one or more snap-fit connections, the snap-fit connections (5.1, 5.2) being mounted in areas of the fluid channel (2) that have a reduced wall thickness (6.1.1, 6.2.1).

2. Adapter (1) according to Claim 1, **characterized in that** the snap-fit connections are structurally bendable and in particular are preferably made of plastic.

3. Adapter (1) according to Claim 2, **characterized in that** the snap-fit connections (5.1, 5.2) are designed as wedge-shaped locking lugs that protrude from the fluid channel (2) preferably at an angle different than 90° and are particularly preferably inclined in the direction of the inlet (3).

4. Adapter (1) according to one of Claims 1-3, **characterized in that** sealing lips (7.2) of circular, oval or rectangular shape are mounted on the adapter in order to close the opening of the medical device in a fluid-tight manner.

5. Adapter (1) according to one of Claims 1-4, **characterized in that** the adapter (1) includes two or more outlets (4.1, 4.2), and preferably at least one outlet (4.2) is oriented transversely with respect to a longitudinal direction of the fluid channel (2) and at least one outlet (4.1) is oriented along the longitudinal direction of the fluid channel (2).

6. Adapter (1) according to one of Claims 1-5, **characterized in that**, if several outlets (4.1, 4.2) are present, the cross-sectional surface areas of the outlet openings are of different sizes, and a ratio of a cross-sectional surface area of a longitudinally oriented outlet opening to a cross-sectional surface area of a transversely oriented outlet opening preferably has a value in the range of 30 - 140.

7. Adapter (1) according to one of Claims 1-6, **characterized in that** a constriction (8) for throttling the stream of fluid through the adapter (1) is mounted in the fluid channel (2).

8. Adapter (1) according to one of Claims 5-7, **characterized in that**, between the outlets (4.1, 4.2), the adapter (1) is provided with protrusions or lips (7.1, 7.2) for dividing the opening (100) of the medical device (50) into parts (101.1, 101.2) separated from each other in a fluid-tight manner.

9. Adapter (1) according to one of Claims 1-8, **characterized in that** a connecting device (9) is mounted in an area of the inlet (3) of the adapter (1), preferably a thread, a component of a bayonet catch, a component of a Luer lock, a clip, or a projection.

10. Adapter (1) according to one of Claims 1-9, **characterized in that** the entire adapter (1) is made of only one material, which is preferably plastic and particularly preferably polypropylene.

11. Adapter (1) according to one of Claims 1-10, **characterized in that** lateral holding projections (10.1, 10.2, 10.3, 10.4) for pulling and pushing are mounted on the adapter (1), making it easier to join and separate the adapter (1) and the medical device (50), and the height of the holding projections (10.1, 10.2, 10.3, 10.4) from the outside of the adapter (1) is preferably more than 4 mm.

12. Set of equipment comprising a medical device (50), an adapter (1) according to one of Claims 1-11, and a fluid container (300), **characterized in that** the inlet (3) of the adapter (1) is connected in a fluid-tight manner to the fluid container (300), which preferably comprises a device (304) for pressure build-up, as are provided in particular in syringes or aerosol cans, and **in that** the outlets (4.1, 4.2) of the adapter (1) are at the same time coupled in a fluid-tight manner to the medical device, which is preferably a dental rotary instrument (50).

13. Use of an adapter (1) according to one of Claims 1-11, **characterized in that**, by way of the inlet (3) of the adapter (1), a fluid (305) is conveyed through the adapter (1) into the channels (104.1, 104.2) of a coupled medical device (50) that are to be cleaned.

## Revendications

1. Adaptateur (1) pour la préparation d'un dispositif technique médical, de préférence d'un instrument dentaire rotatif, comprenant un conduit pour fluide (2) avec une entrée (3) et au moins une sortie (4.1, 4.2), un dispositif d'accouplement (5.1, 5.2) pour une ouverture (100) du dispositif technique médical (50) étant prévu au niveau d'au moins une sortie, **caractérisé en ce que** le dispositif d'accouplement (5.1, 5.2) est réalisé sous forme d'accouplement à usage unique qui, lors du premier enlèvement du dispositif technique médical (50), produit une fuite (200.1, 200.2) dans le conduit de fluide (2) de l'adaptateur (1) et l'accouplement à usage unique étant réalisé sous forme d'une ou plusieurs connexions par encliquetage, les connexions par encliquetage (5.1, 5.2) étant montées dans les régions du conduit de fluide (2) qui présentent une épaisseur de paroi réduite (6.1.1, 6.2.1).

2. Adaptateur (1) selon la revendication 1, **caractérisé en ce que** les connexions par encliquetage sont structurellement flexibles et se composent particulièrement préférablement de plastique.

3. Adaptateur (1) selon la revendication 2, **caractérisé en ce que** les connexions par encliquetage (5.1, 5.2) sont réalisées sous forme de nez d'encliquetage en forme de coin, qui font de préférence saillie suivant un angle différent de 90° depuis le conduit de fluide (2) et qui, de manière particulièrement préférable, sont inclinées dans la direction de l'entrée (3).

4. Adaptateur (1) selon l'une quelconque des revendications 1 à 3, **caractérisé en ce que** des lèvres d'étanchéité (7.2) de forme circulaire, ovale ou rectangulaire sont montées sur l'adaptateur, en vue de fermer de manière étanche aux fluides l'ouverture du dispositif technique médical.

5. Adaptateur (1) selon l'une quelconque des revendications 1 à 4, **caractérisé en ce que** l'adaptateur (1) contient deux ou plusieurs sorties (4.1, 4.2) et de préférence au moins une sortie (4.2) est réalisée transversalement à une direction longitudinale du conduit de fluide (2) et au moins une sortie (4.1) est réalisée le long de la direction longitudinale du conduit de fluide (2).

6. Adaptateur (1) selon l'une quelconque des revendications 1 à 5, **caractérisé en ce que** dans le cas de plusieurs sorties (4.1, 4.2), des surfaces en section transversale des ouvertures de sortie ont des dimensions différentes, un rapport d'une surface en section transversale d'une ouverture de sortie orientée longitudinalement à une surface en section transversale d'une ouverture de sortie orientée transversalement présentant de préférence une valeur de l'ordre de 30 à 140.

7. Adaptateur (1) selon l'une quelconque des revendications 1 à 6, **caractérisé en ce qu'**un rétrécissement (8) pour l'étranglement du jet de fluide à travers l'adaptateur (1) est pratiqué dans le conduit de fluide (2).

8. Adaptateur (1) selon l'une quelconque des revendications 5 à 7, **caractérisé en ce que** des saillies ou des lèvres (7.1, 7.2) sont prévues sur l'adaptateur (1) entre les sorties (4.1, 4.2), afin de diviser l'ouverture (100) du dispositif technique médical (50) en parties séparées les unes des autres de manière étanche aux fluides (101.1, 101.2).

9. Adaptateur (1) selon l'une quelconque des revendications 1 à 8, **caractérisé en ce que** dans une région de l'entrée (3) de l'adaptateur (1) est prévu un dispositif de connexion (9), de préférence un filetage, un composant d'une fermeture par emboîtement à baïonnette, un composant d'une fermeture de Luer, une pince ou une saillie.

10. Adaptateur (1) selon l'une quelconque des revendications 1 à 9, **caractérisé en ce que** l'adaptateur (1) est fabriqué complètement en un seul matériau, qui est de préférence du plastique et particulièrement préférablement du polypropylène.

11. Adaptateur (1) selon l'une quelconque des revendications 1 à 10, **caractérisé en ce que** des saillies de maintien latérales (10.1, 10.2, 10.3, 10.4) sont prévues sur l'adaptateur (1) pour assurer une traction et un aboutement, lesquelles facilitent l'assemblage et la séparation de l'adaptateur (1) et du dispositif technique médical (50), l'avancée des saillies de maintien (10.1, 10.2, 10.3, 10.4) depuis le côté extérieur de l'adaptateur (1) valant de préférence plus de 4 mm.

12. Ensemble d'appareils comprenant un dispositif technique médical (50), un adaptateur (1) selon l'une quelconque des revendications 1 à 11 et un récipient de fluide (300), **caractérisé en ce que** l'entrée (3) de l'adaptateur (1) est connectée de manière étanche aux fluides au récipient de fluide (300), qui dispose de préférence d'un dispositif pour augmenter la pression (304), comme on en prévoit notamment dans des seringues ou des sprays, et **en ce qu'**en même temps les sorties (4.1, 4.2) de l'adaptateur (1) sont accouplées de manière étanche aux fluides au dispositif technique médical, qui est de préférence un instrument dentaire rotatif (50).

13. Utilisation d'un adaptateur (1) selon l'une quelconque des revendications 1 à 11, **caractérisée en ce qu'**un fluide (305) est guidé par le biais de l'entrée (3) de l'adaptateur (1) à travers l'adaptateur (1) dans les conduits (104.1, 104.2) à nettoyer d'un dispositif technique médical (50) raccordé.
